# EUROPEAN PATENT SPECIFICATION

(11) **EP 2 882 475 B1**
(45) Date of publication and mention of the grant of the patent: **13.06.2018**
(21) Application number: 13752869.1
(22) Date of filing: 07.08.2013
(51) Int. Cl.: A61M 5/19, A61M 5/24

(54) **DISPENSE INTERFACE FOR AN EJECTION DEVICE**
AUSGABESCHNITTSTELLE FÜR EINE AUSGABEVORRICHTUNG
INTERFACE DE DISTRIBUTION POUR DISPOSITIF D'ÉJECTION

(30) Priority: 07.08.2012 EP 12179572
(43) Date of publication of application: 17.06.2015
(73) Proprietor: Sanofi-Aventis Deutschland GmbH, 65929 Frankfurt am Main (DE)
(72) Inventor: HOLTWICK, Marc, 65926 Frankfurt am Main (DE); MARX, Wolfgang, 65926 Frankfurt am Main (DE); LAUGERE, Frederic, Shillington Bedfordshire SG5 3NS (GB); POPA, Cristian, South Wootton Norfolk PE30 3RL (GB); IMPEY, Ben, Cambridge Cambridgeshire CB23 7AX (GB); MACLEOD, Andrew, Cambridge Cambridgeshire CB22 7QT (GB)
(74) Representative: McDougall, James
(86) International application number: PCT/EP2013/066555
(87) International publication number: WO 2014/023769

(56) References cited:
- WO-A1-94/26348
- WO-A2-94/22507
- US-A- 5 839 467
- US-A- 6 082 185

## Description

The present patent application relates to a dispense interface for an ejection device, for example a medical device, for delivering at least two liquids, such as liquid drug agents, from separate reservoirs. Such drug agents may comprise a first and a second medicament. The medical device includes a dose setting mechanism for delivering the drug agents automatically or manually by the user.

The medical device can be an injector, for example a hand-held injector, especially a pen-type injector, that is an injector of the kind that provides for administration by injection of medicinal products from one or more multidose cartridges. In particular, the present invention relates to such injectors where a user may set the dose.

The drug agents may be contained in two or more multiple dose reservoirs, containers or packages, each containing independent (single drug compound) or premixed (co-formulated multiple drug compounds) drug agents.

Certain disease states require treatment using one or more different medicaments. Some drug compounds need to be delivered in a specific relationship with each other in order to deliver the optimum therapeutic dose. The present patent application is of particular benefit where combination therapy is desirable, but not possible in a single formulation for reasons such as, but not limited to, stability, compromised therapeutic performance and toxicology.

For example, in some cases it may be beneficial to treat a diabetic with a long acting insulin (also may be referred to as the first or primary medicament) along with a glucagon-like peptide-1 such as GLP-1 or GLP-1 analog (also may be referred to as the second drug or secondary medicament).

Accordingly, there exists a need to provide devices for the delivery of two or more medicaments in a single injection or delivery step that is simple for the user to perform without complicated physical manipulations of the drug delivery device. The proposed drug delivery device provides separate storage containers or cartridge retainers for two or more active drug agents. These active drug agents are then combined and/or delivered to the patient during a single delivery procedure. These active agents may be administered together in a combined dose or alternatively, these active agents may be combined in a sequential manner, one after the other.

The drug delivery device also allows for the opportunity of varying the quantity of the medicaments. For example, one fluid quantity can be varied by changing the properties of the injection device (e.g., setting a user variable dose or changing the device's "fixed" dose). The second medicament quantity can be changed by manufacturing a variety of secondary drug containing packages with each variant containing a different volume and/or concentration of the second active agent.

The drug delivery device may have a single dispense interface. This interface may be configured for fluid communication with a primary reservoir and with a secondary reservoir of medicament containing at least one drug agent. The drug dispense interface can be a type of outlet that allows the two or more medicaments to exit the system and be delivered to the patient.

The combination of compounds from separate reservoirs can be delivered to the body via a double-ended needle assembly. This provides a combination drug injection system that, from a user's perspective, achieves drug delivery in a manner that closely matches the currently available injection devices that use standard needle assemblies. One possible delivery procedure may involve the following steps:
1. Attach a dispense interface to a distal end of the electro-mechanical injection device. The dispense interface comprises a first and a second proximal needle. The first and second needles pierce a first reservoir containing a primary compound and a second reservoir containing a secondary compound, respectively.
2. Attach a dose dispenser, such as a double-ended needle assembly, to a distal end of the dispense interface. In this manner, a proximal end of the needle assembly is in fluidic communication with both the primary compound and secondary compound.
3. Dial up/set a desired dose of the primary compound from the injection device, for example, via a graphical user interface (GUI).
4. After the user sets the dose of the primary compound, the micro-processor controlled control unit may determine or compute a dose of the secondary compound and preferably may determine or compute this second dose based on a previously stored therapeutic dose profile. It is this computed combination of medicaments that will then be injected by the user. The therapeutic dose profile may be user selectable. Alternatively, the user can dial or set a desired dose of the secondary compound.
5. Optionally, after the second dose has been set, the device may be placed in an armed condition. The optional armed condition may be achieved by pressing and/or holding an "OK" or an "Arm" button on a control panel. The armed condition may be provided for a predefined period of time during which the device can be used to dispense the combined dose.
6. Then, the user will insert or apply the distal end of the dose dispenser (e.g. a double ended needle assembly) into the desired injection site. The dose of the combination of the primary compound and the secondary compound (and potentially a third medicament) is administered by activating an injection user interface (e.g. an injection button).

Both medicaments may be delivered via one injection needle or dose dispenser and in one injection step. This offers a convenient benefit to the user in terms of reduced user steps compared to administering two separate injections.

The dispense interfaces in the state of the art are, however, often of complex design. In order to provide the manifold to lead the medicaments from two different reservoirs to a single outlet, multiple complex and/or small parts need to be produced and assembled. In particular, small parts having a complex shape must be molded with a high accuracy in order to provide the delicate channel structures in the dispense interface. The complex part structures and the corresponding complicated assembly steps may cause the dispense interface to be difficult to manufacture and expensive. Additionally, the dispense interface is regularly kept at the drug delivery device for a longer period of time. This means that only the dose dispenser in form of a double-ended needle, for instance, is exchanged for every (or nearly every) injection procedure. The dispense interface, however, remains at the drug delivery device. An exchange of the dispense interface itself is regularly only necessary, when the reservoirs of the drug delivery device need to be exchanged.

This causes the need for a biocompatibility of the dispense interface, which guarantees that either no or negligible amounts of substances can diffuse into drug agents or are set free into the liquid.

Furthermore, if the dispense interface remains attached to the drug delivery device, the different drug agents also start to diffuse into each other over time. A cross-contamination of the drug agents from one reservoir into the other reservoir needs to be prevented for the above mentioned reasons of stability, compromised therapeutic performance and toxicology, for example.

WO 94/26348 discloses a dispense interface according to the preamble of claim 1, and in particular an apparatus for accurately infusing medicinal agents. The apparatus is of a laminate construction and includes an elastic distendable membrane, which in cooperation with a thin planar base defines a fluid chamber having a fluid outlet.

The invention inter-alia faces the technical problem of providing a simple dispense interface for an ejection device that is easy to manufacture.

According to the present invention, there is provided a dispense interface according to claim 1, a method according to claim 10, a system according to claim 12, and a method according to claim 15.

According to a first aspect of the invention, a dispense interface for an ejection device comprises at least two inlets; at least one outlet; a body portion; and a cover film; wherein the body portion and the cover film are bonded together; wherein the body portion and the cover film are configured to form a fluid channel arrangement between surfaces of the body portion and the cover film facing each other when the body portion and the cover film are bonded together; and wherein the fluid channel arrangement is configured to provide fluid communication between the at least two inlets and the at least one outlet.

The ejection device may be a drug delivery device such as a medical device configured to eject a drug agent (e.g. a dose of a medicament) such as an infusion device or an injection device, for instance an insulin injection pen. Injection devices may be used either by medical personnel or by patients themselves. As an example, type-1 and type-2 diabetes may be treated by patients themselves by injection of insulin doses, for example once or several times per day. In particular, the ejection device may be a medical device configured to deliver (e.g. eject) at least two drug agents from separate reservoirs.

Alternatively, the ejection device may for instance be configured to deliver (e.g. eject) a two-component adhesive from separate fluid reservoirs comprising a first component of the two-component adhesive (e.g. a binder) and a second component of the two-component adhesive (e.g. a hardener), respectively.

The dispense interface may be a disposable part attachable to the ejection device (e.g. the medical device). In particular, the dispense interface may be a single-use part attachable to the ejection device. Each of the at least two inlets of the dispense interface may be configured to reside in fluid communication with one of at least two separate fluid reservoirs of the ejection device when the dispense interface is attached to the ejection device.

The ejection device and/or the dispense interface may preferably be portable (e.g. handheld) devices.

The fluid channel arrangement may provide a fluid connection between each of the at least two inlets of the dispense interface and the at least one outlet of the dispense interface. Also, the fluid channel arrangement may provide a fluid connection between the at least two inlets of the dispense interface. For instance, the fluid channel arrangement is at least partially Y-like, T-like or Z-like shaped.

The fluid channel arrangement may comprise one or more connected fluid channels. The cross-section of the fluid channels may be circular, semi-circular, rectangular, V-shaped (triangular), or any other shape that is easily manufacturable and/or suitable for providing a fluid connection. The diameter of the fluid channels may be between 0.01 mm and 10mm. In particular, the diameter of the fluid channels may be between 0.1mm and 1mm. For instance, the fluid channel may have a semi-circular cross-section and a diameter of about 0.3mm. The ratio between the length of the fluid channel arrangement and the width of the fluid channel (length:width ratio), for example represented by the diameter of the fluid channels providing a length:diameter ratio, may be substantially large, for instance between 10:1 and 1000:1. In particular, the length:width ratio (or length:diameter ratio) may be between 20:1 and 100:1, for instance about 33:1 or 66:1. The length of the fluid channel arrangement may preferably describe the longest fluid path of the fluid channel arrangement.

Integrally formed parts having a fluid channel arrangement with a substantially large ratio between the length of the fluid channel arrangement and the width or diameter of the fluid channels cannot be simply manufactured, for instance by molding such as injection molding. This is inter-alia due to the fact that the fluid channel arrangement is difficult to access. Complex tooling is necessary to manufacture such parts.

Since the fluid channel arrangement of the dispense interface of the first aspect of the invention is formed between surfaces of the body portion and the cover film facing each other when the body portion and the cover film are bonded with each other, both the body portion and the cover film provide a part of the walls of the fluid channel arrangement. The surfaces of the body portion and the cover film are easily accessible. Therefore, manufacturing thereof is simplified.

The body portion may for instance be manufactured by molding such as injection molding, for instance by use of an open-and-shut tool without the need for complex tooling. By joining the body portion and the cover film after manufacturing thereof, it is thus possible to form a joined part having fluid channels with a large length:width ratio and/or a complex geometry and/or tight tolerances.

The fluid channel arrangement may at least substantially be arranged in a sectional plane of the dispense interface. The sectional plane may be a vertical plane of the dispense interface and/or a longitudinal plane of the dispense interface. Preferably, the fluid channel arrangement should be understood to be at least substantially arranged in a sectional plane of the dispense interface if at least a predominant portion of the fluid channel arrangement (e.g. more than 50%, preferably more than 75%, and more preferably more than 90%) is cut by the sectional plane of the dispense interface.

In addition to the fluid channel arrangement formed between the surfaces of the body portion and the cover film facing each other when the body portion and the cover film are bonded together, the body portion may comprise further fluid channels.

The at least one outlet of the dispense interface may serve as a common outlet for separate fluid reservoirs of the ejection device. As described above, each of these separate fluid reservoirs may reside in fluid communication with one of the at least two inlets of the dispense interface when the dispense interface is attached to the ejection device.

The at least two inlets of the dispense interface and/or the at least one outlet of the dispense interface may be arranged in the body portion and/or between the surfaces of the body portion and the cover film. For instance, the at least two inlets of the dispense interface and/or the at least one outlet of the dispense interface may be formed integrally with the body portion.

The cover film bonded to the body portion may be a flexible film. It may thus balance any unevenness or curvature of the surface of the body portion. The cover film may in particular be a foil or a laminate consisting of two or more layers of different or the same material. For instance, the thickness of the cover film may be 1 µm to 1 mm, in particular 5 µm to 500 µm. The cover film may also consist of multiple cover film parts or sections, each bonded to the body portion.

The bonding of the cover film to the body portion may be achieved by adhesive and/or thermal bonding techniques to provide non-limiting examples. For adhesive bonding an adhesive, in particular an adhesive having bio-compatibility, may be applied to the surface of the body portion and/or of the cover film facing each other when bonded. The adhesive may be based on a polymer. The adhesive may be an organic or inorganic adhesive. Alternatively or additionally, thermal bonding may be utilized to bond the surfaces of the body portion and the film cover facing each other. A non-limiting example is the fusion welding technique. During fusion welding the materials of the body portion and/or the cover film are melted in order to join the body portion with the cover film. For thermal bonding laser welding may be applied to name another example. A custom and precise heat-affected zone may be produced in this way. Thermal bonding is inter alia advantageous, since no adhesive is needed and thus the number of materials in contact with the liquid can be reduced.

Due to the arrangement of the fluid channel arrangement between the surfaces of the body portion and the cover film the fluid channel arrangement may be provided in a single manufacturing step of bonding of the body portion and the cover film. Furthermore, the count and complexity of assembly parts of the dispense interface is reduced. The invention is therefore inter alia advantageous to allow a simple manufacturing and/or assembly of a dispense interface. Also, it allows a cost-effective manufacturing/assembly of a disposable dispense interface (e.g. a single-use dispense interface).

According to an exemplary embodiment of the dispense interface of the first aspect of the invention, the cover film is a metal film, a polymer film, or a bio-polymer film. The metal film may in particular be an aluminium foil. Bio-polymer materials, i.e. polymer materials that are biocompatible, are, for instance, COP (cyclo-olefin polymer) materials, which may be used for production of the cover film. COP materials have a high biocompatibility. For instance, COP materials have little to no extractables and most COP material can undergo sterilization by gamma radiation, steam and/or ethylene oxide. Other polymer materials such as PP (poly-propylene) or HDPE (high density poly-ethylene) or other less expensive materials may be used, too. Especially, a single use dispense interface may be made from such a material, as the contact time with the medicament is rather short (only the time from priming the device until the injection is completed).

As described above, the cover film may also be a laminate, for example an aluminium laminate, a polymer laminate, a bio-polymer laminate or comprise combinations thereof. Different layers of the above materials may also be combined, for example. It may be advantageous, if the layer of the laminate, which is in contact with the surface of the body portion, is a bio-polymer, to increase bio-compatibility.

According to an exemplary embodiment of the dispense interface of the first aspect of the invention, the body portion is manufactured from a polymer material, in particular a bio-polymer.

Polymer materials may, in particular, be used for the production of the body portion via molding, e.g. injection molding. Polymer materials are specifically suitable for molding. As described above with respect to the cover film, polymer materials are typically biocompatible. For instance, COP materials having a high biocompatibility may be used for the production of the body portion, as well. However, other materials or polymers such as PP (poly-propylene) or HDPE (high density poly-ethylene) or other less expensive materials may be used for the body portion, as well. Particularly, a single use dispense interface may be made from such a less expensive material, as the contact time with the medicament is rather short.

According to an exemplary embodiment of the dispense interface of the first aspect of the invention, each of the at least two inlets is formed from a fluid connector emptying into the fluid channel arrangement, and wherein each of the fluid connectors is configured to establish a releasable fluid connection with a corresponding fluid connector of a fluid reservoir of the ejection device when the dispense interface is attached to the ejection device.

Non-limiting examples of a fluid connector may be a piercing needle, a piercable septum and/or a (male/female) Luer-connector. Such a fluid connector may be integrally formed with the body portion. Alternatively, such a fluid connector may at least partially be inserted (e.g. potted/over-molded/mounted) into the body portion. For instance, such a fluid connector may at least partially be potted/over-molded when the body portion is (e.g. injection) molded. For instance, such a fluid connector may at least partially be inserted (e.g. glued/mounted) in a separate step after the body portion has been produced, for example molded.

According to an exemplary embodiment of the dispense interface of the first aspect of the invention, the at least one outlet is formed from a fluid connector, wherein the fluid channel arrangement empties into the fluid connector, and wherein the fluid connector is configured to establish a fluid connection with a corresponding fluid connector of a (ejection) needle assembly, when the needle assembly is attached to the dispense interface. The needle assembly may have an injection needle for penetrating the skin of a patient.

As described above, non-limiting examples of a fluid connector may be a piercing needle, a piercable septum and/or a (male/female) Luer-connector. Such a fluid connector may be integrally formed with the body portion. Alternatively, such a fluid connector may at least partially be inserted (e.g. potted/over-molded/mounted) into the body portion. For instance, such a fluid connector may at least partially be potted/over-molded when the body portion is (e.g. injection) molded. For instance, such a fluid connector may at least partially be inserted (e.g. glued/mounted) in a separate step after the body portion has been produced, for example molded.

The fluid connector forming the at least one outlet of the dispense interface, allows to exchange the needle assembly more often than the dispense interface. This is inter-alia advantageous if the needle assembly is a single-use device which has to be replaced after a single ejection and the dispense interface is a disposable part which can be used for more than one ejection.

According to an exemplary embodiment of the dispense interface of the first aspect of the invention, the at least one outlet is formed from a needle, wherein the fluid channel arrangement empties into the needle. The needle may be an injection needle for penetrating the skin of a patient such as a cannula.

The needle may at least partially be inserted (e.g. potted/over-molded/mounted) into the body portion. For instance, the needle may at least partially be potted/over-molded when the body portion is molded (e.g. injection molded). For instance, the needle may at least partially be glued/mounted in a separate step after the body portion has been (injection) molded. For instance, the needle may be an integral part of the dispense interface.

Since the at least one outlet is already formed from a needle, no attachment of a separate needle assembly is necessary. This embodiment thus inter-alia allows to reduce the overall complexity of the dispense interface and/or the ejection device. This is inter-alia advantageous if the dispense interface is a single use device which has to be replaced after a single ejection.

According to the invention, fluid grooves are arranged in the surface of the body portion facing the cover film and the surface of the cover film seals the fluid grooves when the body portion and the cover film are bonded together.

The fluid grooves may be any indentations on the surface of the body portion which permits the passing of fluid along the surface thereof. The surface of the cover film seal the fluid grooves when the body portion and the film cover are bonded to each other such that a tight fluid channel arrangement is formed.

The fluid grooves arranged in the surface of the body portion facing the cover film may be of any desired shape in their cross section perpendicular to the flow direction, for example of part-circular or semi-circular shape, of polygonal shape, of rectangular shape or of combinations thereof in sections. In case the body portion is produced by (injection) molding, the fluid grooves may be provided during the molding process. In other words, a body portion with open channels molded into the surface is provided. Alternatively, the body portion may first be produced without fluid grooves as a base plate. The fluid grooves may then be molded or carved into the base plate to provide the body portion. Additionally, it is also conceivable, that fluid grooves may be arranged in the surface of the cover film, wherein the surface of the body portion may be configured to cover these fluid grooves when the body portion and the cover film are bonded together.

According to an exemplary embodiment of the dispense interface of the first aspect of the invention, the dispense interface further comprises at least one ullage; wherein the ullage provides fluid communication between an adjacent fluid groove and an adjacent inlet or outlet.

The at least one ullage may be provided as an open recess on the surface of the body portion facing the cover film. An ullage is in particular understood to provide a larger diameter of the fluid channel arrangement than the fluid grooves. The at least one ullage may be sealed by the cover film similar to the fluid grooves, for example. The at least one ullage may be produced during (injection) molding of the body portion or may be molded or carved into the body portion after molding, for example. Preferably, there is provided an ullage for each inlet and/or outlet. The connection of fluid connectors may be facilitated since the respective ullage provides a larger diameter than the fluid grooves and thus a larger tolerance is provided for the insertion of the fluid connectors into the body portion. Additionally or alternatively, the at least one ullage may provide room for the implementation of a valve arrangement within the ullage.

According to an exemplary embodiment of the dispense interface of the first aspect of the invention, the dispense interface further comprises a valve arrangement configured to control a fluid flow from the at least two inlets to the at least one outlet via the fluid channel arrangement. The valve arrangement may comprise one or more valves, preferably one or more non-return valves. Such a valve arrangement may preferably be configured to prevent cross contamination of fluids contained in separate fluid reservoirs of the ejection device. A preferred valve arrangement may also be configured so as to prevent back flow. Non-limiting examples of such valves are a diaphragm/flap valve, a shuttling valve, a molded duck bill valve, a flat spring valve and/or a rotating flap valve.

The valve arrangement may for instance be integrally formed with the body portion. Alternatively, the valve arrangement may for instance be manufactured separately from the body portion. The valve arrangement may be inserted (e.g. potted/over-molded/mounted) into the body portion, in particular into one or more ullages. For instance, the valve arrangement may at least partially be potted/over-molded when the body portion is (e.g. injection) molded. For instance, the valve arrangement may at least partially be mounted in a separate step after the body portion has been (injection) molded. After provision of the valve arrangement in the body portion the cover film may seal the fluid grooves and thus the fluid channel arrangement. According to a second aspect of the invention, a method for manufacturing a dispense interface of the first aspect of the invention comprises molding fluid grooves into a surface of a body portion; bonding a cover film to the body portion, such that a fluid channel arrangement is formed between surfaces of the body portion and the cover film facing each other when the body portion and the cover film are bonded together, and wherein wherein the surface of the cover film seals the fluid grooves when the body portion and the cover film are bonded together. The fluid grooves may be molded into the body portion during molding, for example injection molding, of the body portion, or the fluid grooves may be molded and/or carved into the body portion after production of the body portion. According to an exemplary embodiment of the method of the second aspect of the invention, the method further comprises connecting fluid connectors forming the at least two inlets to the body portion in a sealing manner. The connection may be performed before or after the bonding of the cover film to the body portion. The connection may be performed during or after molding of the body portion by means of over-molding/insert-molding or potting, for example.

Additionally or alternatively, the at least one fluid connector forming the at least one outlet can be connected to the body part in a corresponding manner.

According to a third aspect of the invention, a system comprises the dispense interface of the first aspect of the invention and an ejection device; wherein the dispense interface is attached to the ejection device. The system may further comprise a needle assembly, wherein the needle assembly is attached to the dispense interface. The dispense interface may provide a fluid connection between at least two separate fluid reservoirs of the ejection device and the needle assembly. As described above, the ejection device may be a medical device configured to deliver (e.g. eject) a medicament.

According to a fourth aspect of the invention, a method for using the system of the third aspect of the invention, excluding in-vivo applications, comprises attaching the dispense interface to an ejection device having at least two fluid reservoirs; ejecting a fluid from at least one of the reservoirs through the dispense interface; and detaching the dispense interface from the ejection device. The method may furthermore comprise attaching a needle assembly to a dispense interface, wherein the fluid is ejected from at least one of the reservoirs through the dispense interface out of the needle assembly.

Exemplary features/embodiments (exhibiting further features) of the invention have been described above, which are understood to apply to the various aspects of the invention. These single features/embodiments are considered to be exemplary and non-limiting, and to be respectively combinable independently from other disclosed features of the various aspects of the invention as described above. Nevertheless, these exemplary features/embodiments shall also be considered to be disclosed in all possible combinations with each other and with the various aspects of the invention as described above.

These as well as other advantages of various aspects of the present invention will become apparent to those of ordinary skill in the art by reading the following detailed description, with appropriate reference to the accompanying drawings, in which:
- Fig. 1: illustrates a perspective view of a delivery device with an end cap of the device removed;
- Fig. 2: illustrates a perspective view of the delivery device distal end showing the cartridge;
- Fig. 3: illustrates a perspective view of the delivery device illustrated in Fig. 1 or 2 with one cartridge retainer in an open position;
- Fig. 4: illustrates a comparative dispense interface and a dose dispenser that may be removably mounted on a distal end of the delivery device illustrated in Fig. 1;
- Fig. 5: illustrates the dispense interface and the dose dispenser illustrated in Fig. 4 mounted on a distal end of the delivery device illustrated in Fig. 1;
- Fig. 6: illustrates one arrangement of a needle assembly that may be mounted on a distal end of the delivery device;
- Fig. 7: illustrates a perspective view of the dispense interface illustrated in Fig. 4;
- Fig. 8: illustrates another perspective view of the dispense interface illustrated in Fig. 4;
- Fig. 9: illustrates a cross-sectional view of the dispense interface illustrated in Fig. 4;
- Fig. 10: illustrates an exploded view of the dispense interface illustrated in Fig. 4;
- Fig. 11: illustrates a cross-sectional view of the comparative dispense interface and needle assembly mounted onto a drug delivery device, such as the device illustrated in Fig. 1;
- Fig. 12a: illustrates a perspective view of an embodiment of a dispense interface before bonding the cover film to the body portion;
- Fig. 12b: illustrates an enlarged cross sectional view of the body portion of Fig. 12a;
- Fig. 13a: illustrates a perspective view of the dispense interface from Fig. 12a after bonding the cover film to the body portion;
- Fig. 13b: illustrates an enlarged cross sectional view of the dispense interface from Fig. 13a;
- Fig. 14a: illustrates an alternative embodiment of a valve arrangement of a dispense interface;
- Fig. 14b: illustrates another alternative embodiment of a valve arrangement of a dispense interface;
- Fig. 14c: illustrates another alternative embodiment of a valve arrangement of a dispense interface;
- Fig. 14d: illustrates another alternative embodiment of a valve arrangement of a dispense interface; and
- Fig. 14e: illustrates another alternative embodiment of a valve arrangement of a dispense interface.
- Fig. 15: illustrates a flowchart of a method according to the invention for using a dispense interface.

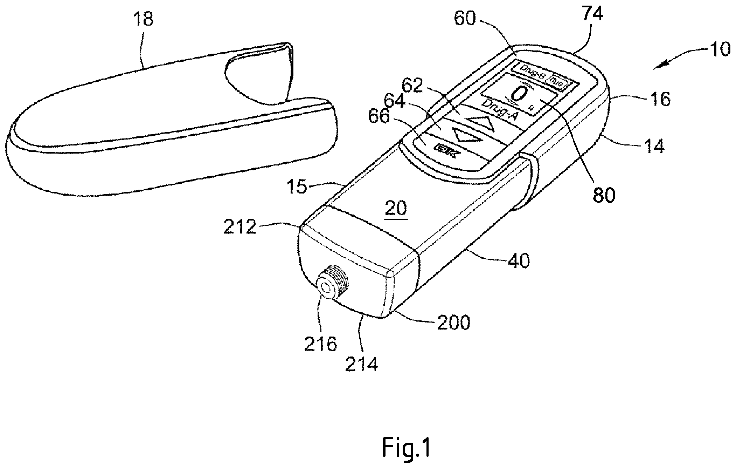

An ejection device in form of a drug delivery device illustrated in Fig. 1 comprises a main body 14 that extends from a proximal end 16 to a distal end 15. At the distal end 15, a removable end cap or cover 18 is provided. This end cap 18 and the distal end 15 of the main body 14 work together to provide a snap fit or form fit connection so that once the cover 18 is slid onto the distal end 15 of the main body 14, this frictional fit between the cap and the main body outer surface 20 prevents the cover from inadvertently falling off the main body.

The main body 14 contains a micro-processor control unit, an electro-mechanical drive train, and at least two medicament reservoirs. When the end cap or cover 18 is removed from the device 10 (as illustrated in Fig. 1), a comparative dispense interface 200 is mounted to the distal end 15 of the main body 14, and a dose dispenser (e.g., a needle assembly) is attached to the interface. The drug delivery device 10 can be used to administer a computed dose of a second medicament (secondary drug compound) and a variable dose of a first medicament (primary drug compound) through a single needle assembly, such as a double ended needle assembly.

The drive train may exert a pressure on the bung of each cartridge, respectively, in order to expel the doses of the first and second medicaments. For example, a piston rod may push the bung of a cartridge forward a pre-determined amount for a single dose of medicament. When the cartridge is empty, the piston rod is retracted completely inside the main body 14, so that the empty cartridge can be removed and a new cartridge can be inserted.

A control panel region 60 is provided near the proximal end of the main body 14. Preferably, this control panel region 60 comprises a digital display 80 along with a plurality of human interface elements that can be manipulated by a user to set and inject a combined dose. In this arrangement, the control panel region comprises a first dose setting button 62, a second dose setting button 64 and a third button 66 designated with the symbol "OK." In addition, along the most proximal end of the main body, an injection button 74 is also provided (not visible in the perspective view of Fig. 1). The user interface of the drug delivery device may comprise additional buttons, such as a "menu" button, a "back" button, or a "light" button to switch on an illumination of the display.

The cartridge holder 40 can be removably attached to the main body 14 and may contain at least two cartridge retainers 50 and 52. Each retainer is configured so as to contain one medicament reservoir, such as a glass cartridge. Preferably, each cartridge contains a different medicament.

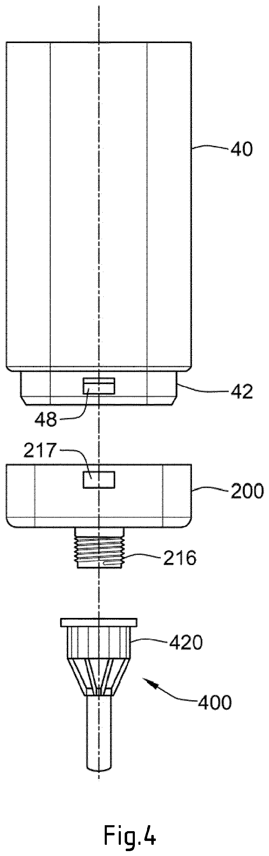

In addition, at the distal end of the cartridge holder 40, the drug delivery device illustrated in Fig. 1 includes a dispense interface 200. As will be described in relation to Fig. 4, in one arrangement, this dispense interface 200 includes a main outer body 212 that is removably attached to a distal end 42 of the cartridge housing 40. As can be seen in Fig. 1, a distal end 214 of the dispense interface 200 preferably comprises a needle hub 216. This needle hub 216 may be configured so as to allow a dose dispenser, such as a conventional pen type injection needle assembly, to be removably mounted to the drug delivery device 10.

Once the device is turned on, the digital display 80 shown in Fig. 1 illuminates and provides the user certain device information, preferably information relating to the medicaments contained within the cartridge holder 40. For example, the user is provided with certain information relating to both the primary medicament (Drug A) and the secondary medicament (Drug B).

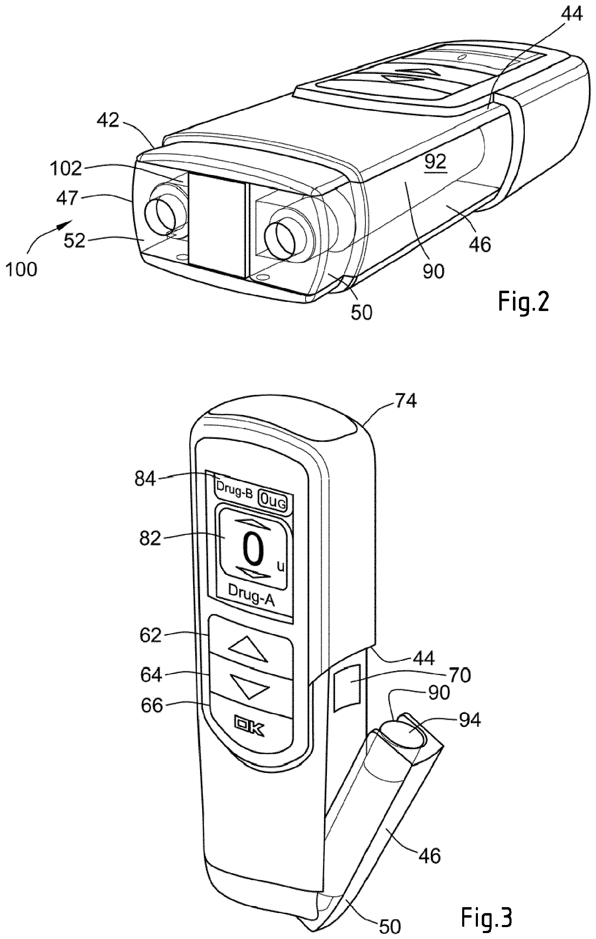

As shown in Fig. 3, the first and second cartridge retainers 50, 52 may be hinged cartridge retainers. These hinged retainers allow user access to the cartridges. Fig. 3 illustrates a perspective view of the cartridge holder 40 illustrated in Fig. 1 with the first hinged cartridge retainer 50 in an open position. Fig. 3 illustrates how a user might access the first cartridge 90 by opening up the first retainer 50 and thereby having access to the first cartridge 90.

As mentioned above when discussing Fig. 1, a dispense interface 200 can be coupled to the distal end of the cartridge holder 40. Fig. 4 illustrates a flat view of the dispense interface 200 unconnected to the distal end of the cartridge holder 40. A dose dispenser or needle assembly 400 that may be used with the interface 200 is also illustrated and is provided in a protective outer cap 420.

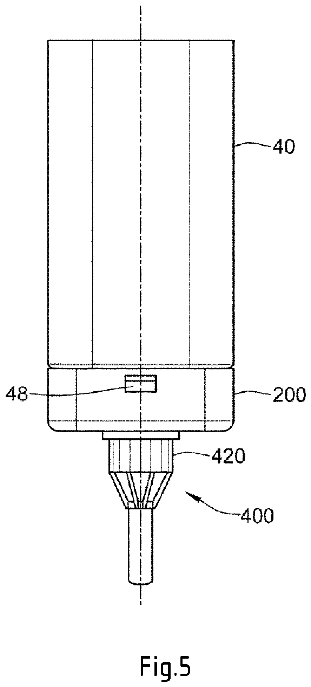

In Fig. 5, the dispense interface 200 illustrated in Fig. 4 is shown coupled to the cartridge holder 40. The axial attachment means 48 between the dispense interface 200 and the cartridge holder 40 can be any known axial attachment means to those skilled in the art, including snap locks, snap fits, snap rings, keyed slots, and combinations of such connections. The connection or attachment between the dispense interface and the cartridge holder may also contain additional features (not shown), such as connectors, stops, splines, ribs, grooves, pips, clips and the like design features, that ensure that specific hubs are attachable only to matching drug delivery devices. Such additional features would prevent the insertion of a non-appropriate secondary cartridge to a non-matching injection device.

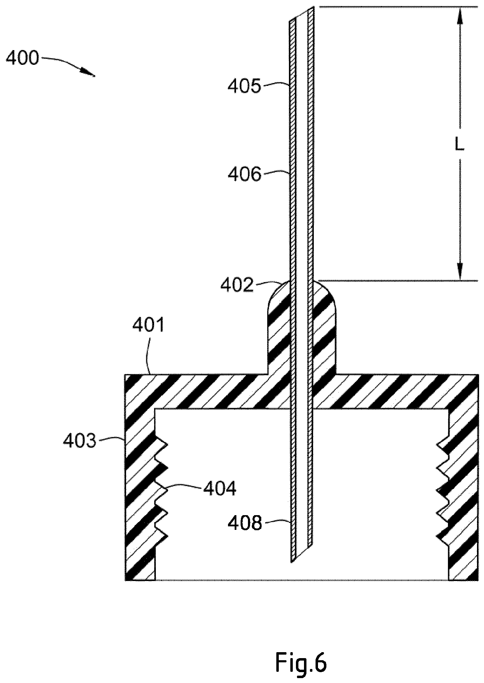

Fig. 5 also illustrates the needle assembly 400 and protective cover 420 coupled to the distal end of the dispense interface 200 that may be screwed onto the needle hub of the interface 200. Fig. 6 illustrates a cross sectional view of the double ended needle assembly 400 mounted on the dispense interface 200 in Fig. 5.

The needle assembly 400 illustrated in Fig. 6 comprises a double ended needle 406 and a hub 401. The double ended needle or cannula 406 is fixedly mounted in a needle hub 401. This needle hub 401 comprises a circular disk shaped element which has along its periphery a circumferential depending sleeve 403. Along an inner wall of this hub member 401, a thread 404 is provided. This thread 404 allows the needle hub 401 to be screwed onto the dispense interface 200 which, in one preferred arrangement, is provided with a corresponding outer thread along a distal hub. At a center portion of the hub element 401 there is provided a protrusion 402. This protrusion 402 projects from the hub in an opposite direction of the sleeve member. A double ended needle 406 is mounted centrally through the protrusion 402 and the needle hub 401. This double ended needle 406 is mounted such that a first or distal piercing end 405 of the double ended needle forms an injecting part for piercing an injection site (e.g., the skin of a user).

Similarly, a second or proximal piercing end 408 of the needle assembly 400 protrudes from an opposite side of the circular disc so that it is concentrically surrounded by the sleeve 403. In one needle assembly arrangement, the second or proximal piercing end 408 may be shorter than the sleeve 403 so that this sleeve to some extent protects the pointed end of the back sleeve. The needle cover cap 420 illustrated in Fig. 4 and 5 provides a form fit around the outer surface 403 of the hub 401.

Referring now to Fig. 4 to 11, one preferred arrangement of this interface 200 will now be discussed. In this one preferred arrangement, this interface 200 comprises:
a. a main outer body 210,
b. an first inner body 220,
c. a second inner body 230,
d. a first piercing needle 240,
e. a second piercing needle 250,
f. a valve seal 260, and
g. a septum 270.

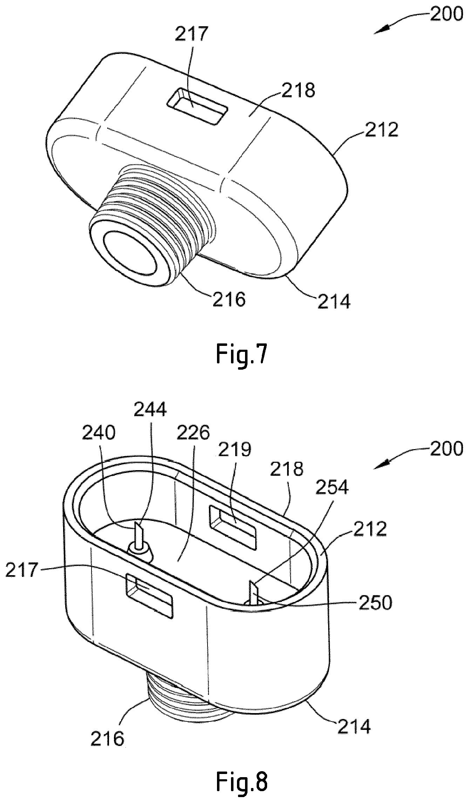

The main outer body 210 comprises a main body proximal end 212 and a main body distal end 214. At the proximal end 212 of the outer body 210, a connecting member is configured so as to allow the dispense interface 200 to be attached to the distal end of the cartridge holder 40. Preferably, the connecting member is configured so as to allow the dispense interface 200 to be removably connected the cartridge holder 40. In one preferred interface arrangement, the proximal end of the interface 200 is configured with an upwardly extending wall 218 having at least one recess. For example, as may be seen from Fig. 8, the upwardly extending wall 218 comprises at least a first recess 217 and a second recess 219.

Preferably, the first and the second recesses 217, 219 are positioned within this main outer body wall so as to cooperate with an outwardly protruding member located near the distal end of the cartridge housing 40 of the drug delivery device 10. For example, this outwardly protruding member 48 of the cartridge housing may be seen in Fig. 4 and 5. A second similar protruding member is provided on the opposite side of the cartridge housing. As such, when the interface 200 is axially slid over the distal end of the cartridge housing 40, the outwardly protruding members will cooperate with the first and second recess 217, 219 to form an interference fit, form fit, or snap lock. Alternatively, and as those of skill in the art will recognize, any other similar connection mechanism that allows for the dispense interface and the cartridge housing 40 to be axially coupled could be used as well.

The main outer body 210 and the distal end of the cartridge holder 40 act to form an axially engaging snap lock or snap fit arrangement that could be axially slid onto the distal end of the cartridge housing. In one alternative arrangement, the dispense interface 200 may be provided with a coding feature so as to prevent inadvertent dispense interface cross use. That is, the inner body of the hub could be geometrically configured so as to prevent an inadvertent cross use of one or more dispense interfaces.

A mounting hub is provided at a distal end of the main outer body 210 of the dispense interface 200. Such a mounting hub can be configured to be releasably connected to a needle assembly. As just one example, this connecting means 216 may comprise an outer thread that engages an inner thread provided along an inner wall surface of a needle hub of a needle assembly, such as the needle assembly 400 illustrated in Fig. 6. Alternative releasable connectors may also be provided such as a snap lock, a snap lock released through threads, a bayonet lock, a form fit, or other similar connection arrangements.

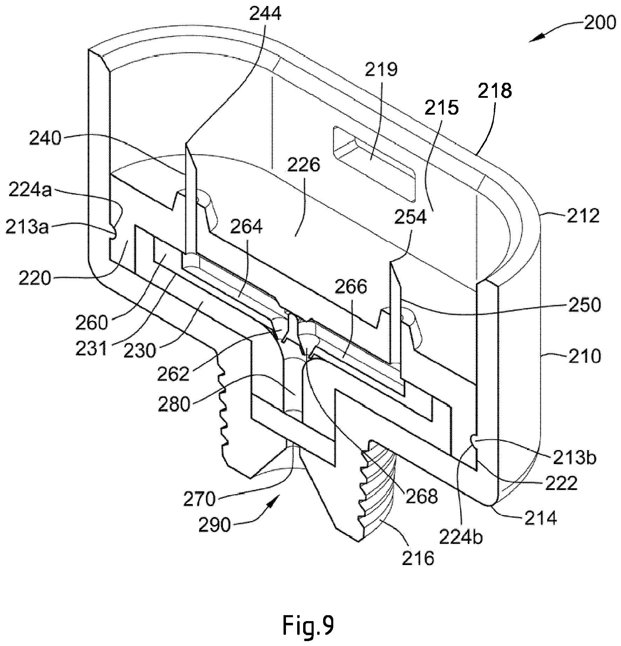

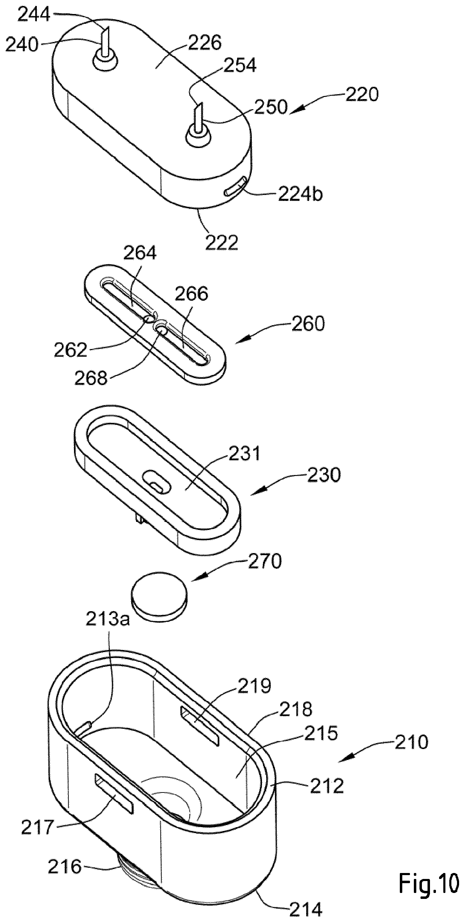

The dispense interface 200 further comprises a first inner body 220. Certain details of this inner body are illustrated in Fig. 8-11. Preferably, this first inner body 220 is coupled to an inner surface 215 of the extending wall 218 of the main outer body 210. More preferably, this first inner body 220 is coupled by way of a rib and groove form fit arrangement to an inner surface of the outer body 210. For example, as can be seen from Fig. 9, the extending wall 218 of the main outer body 210 is provided with a first rib 213a and a second rib 213b. This first rib 213a is also illustrated in Fig. 10. These ribs 213a and 213b are positioned along the inner surface 215 of the wall 218 of the outer body 210 and create a form fit or snap lock engagement with cooperating grooves 224a and 224b of the first inner body 220. In a preferred arrangement, these cooperating grooves 224a and 224b are provided along an outer surface 222 of the first inner body 220.

In addition, as can be seen in Fig. 8-10, a proximal surface 226 near the proximal end of the first inner body 220 may be configured with at least a first proximally positioned piercing needle 240 comprising a proximal piercing end portion 244. Similarly, the first inner body 220 is configured with a second proximally positioned piercing needle 250 comprising a proximally piercing end portion 254. Both the first and second needles 240, 250 are rigidly mounted on the proximal surface 226 of the first inner body 220.

Preferably, this dispense interface 200 further comprises a valve arrangement. Such a valve arrangement could be constructed so as to prevent cross contamination of the first and second medicaments contained in the first and second reservoirs, respectively. A preferred valve arrangement may also be configured so as to prevent back flow and cross contamination of the first and second medicaments.

In one preferred system, dispense interface 200 includes a valve arrangement in the form of a valve seal 260. Such a valve seal 260 may be provided within a cavity 231 defined by the second inner body 230, so as to form a holding chamber 280. Preferably, cavity 231 resides along an upper surface of the second inner body 230. This valve seal comprises an upper surface that defines both a first fluid groove 264 and second fluid groove 266. For example, Fig. 9 illustrates the position of the valve seal 260, seated between the first inner body 220 and the second inner body 230. During an injection step, this seal valve 260 helps to prevent the primary medicament in the first pathway from migrating to the secondary medicament in the second pathway, while also preventing the secondary medicament in the second pathway from migrating to the primary medicament in the first pathway. Preferably, this seal valve 260 comprises a first non-return valve 262 and a second non-return valve 268. As such, the first non-return valve 262 prevents fluid transferring along the first fluid pathway 264, for example a groove in the seal valve 260, from returning back into this pathway 264. Similarly, the second non-return valve 268 prevents fluid transferring along the second fluid pathway 266 from returning back into this pathway 266.

Together, the first and second grooves 264, 266 converge towards the non-return valves 262 and 268 respectively, to then provide for an output fluid path or a holding chamber 280. This holding chamber 280 is defined by an inner chamber defined by a distal end of the second inner body both the first and the second non return valves 262, 268 along with a pierceable septum 270. As illustrated, this pierceable septum 270 is positioned between a distal end portion of the second inner body 230 and an inner surface defined by the needle hub of the main outer body 210.

The holding chamber 280 terminates at an outlet port of the interface 200. This outlet port 290 is preferably centrally located in the needle hub of the interface 200 and assists in maintaining the pierceable seal 270 in a stationary position. As such, when a double ended needle assembly is attached to the needle hub of the interface (such as the double ended needle illustrated in Fig. 6), the output fluid path allows both medicaments to be in fluid communication with the attached needle assembly.

The hub interface 200 further comprises a second inner body 230. As can be seen from Fig. 9, this second inner body 230 has an upper surface that defines a recess, and the valve seal 260 is positioned within this recess. Therefore, when the interface 200 is assembled as shown in Fig. 9, the second inner body 230 will be positioned between a distal end of the outer body 210 and the first inner body 220. Together, second inner body 230 and the main outer body hold the septum 270 in place. The distal end of the inner body 230 may also form a cavity or holding chamber that can be configured to be fluid communication with both the first groove 264 and the second groove 266 of the valve seal.

Axially sliding the main outer body 210 over the distal end of the drug delivery device attaches the dispense interface 200 to the multi-use device. In this manner, a fluid communication may be created between the first needle 240 and the second needle 250 with the primary medicament of the first cartridge and the secondary medicament of the second cartridge, respectively.

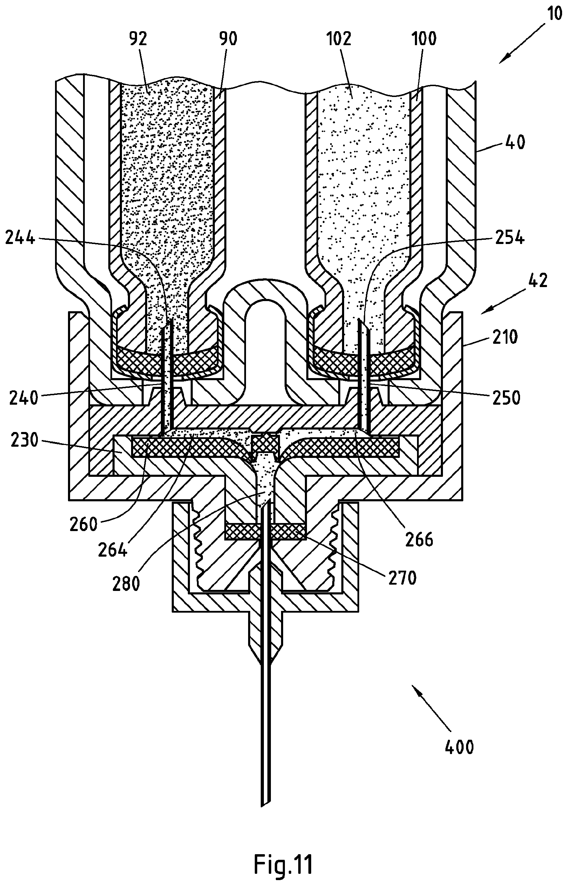

Fig. 11 illustrates the dispense interface 200 after it has been mounted onto the distal end 42 of the cartridge holder 40 of the drug delivery device 10 illustrated in Fig. 1. A double ended needle 400 is also mounted to the distal end of this interface. The cartridge holder 40 is illustrated as having a first cartridge containing a first medicament and a second cartridge containing a second medicament.

When the interface 200 is first mounted over the distal end of the cartridge holder 40, the proximal piercing end 244 of the first piercing needle 240 pierces the septum of the first cartridge 90 and thereby resides in fluid communication with the primary medicament 92 of the first cartridge 90. A distal end of the first piercing needle 240 will also be in fluid communication with a first fluid path groove 264 defined by the valve seal 260.

Similarly, the proximal piercing end 254 of the second piercing needle 250 pierces the septum of the second cartridge 100 and thereby resides in fluid communication with the secondary medicament 102 of the second cartridge 100. A distal end of this second piercing needle 250 will also be in fluid communication with a second fluid path groove 266 defined by the valve seal 260.

Fig. 11 illustrates a preferred arrangement of such a dispense interface 200 that is coupled to a distal end 15 of the main body 14 of drug delivery device 10. Preferably, such a dispense interface 200 is removably coupled to the cartridge holder 40 of the drug delivery device 10.

As illustrated in Fig. 11, the dispense interface 200 is coupled to the distal end of a cartridge housing 40. This cartridge holder 40 is illustrated as containing the first cartridge 90 containing the primary medicament 92 and the second cartridge 100 containing the secondary medicament 102. Once coupled to the cartridge housing 40, the dispense interface 200 essentially provides a mechanism for providing a fluid communication path from the first and second cartridges 90, 100 to the common holding chamber 280. This holding chamber 280 is illustrated as being in fluid communication with a dose dispenser. Here, as illustrated, this dose dispenser comprises the double ended needle assembly 400. As illustrated, the proximal end of the double ended needle assembly is in fluid communication with the chamber 280. In one preferred arrangement, the dispense interface is configured so that it attaches to the main body in only one orientation, that is it is fitted only one way round. As such as illustrated in Fig. 11, once the dispense interface 200 is attached to the cartridge holder 40, the primary needle 240 can only be used for fluid communication with the primary medicament 92 of the first cartridge 90 and the interface 200 would be prevented from being reattached to the holder 40 so that the primary needle 240 could now be used for fluid communication with the secondary medicament 102 of the second cartridge 100. Such a one way around connecting mechanism may help to reduce potential cross contamination between the two medicaments 92 and 102.

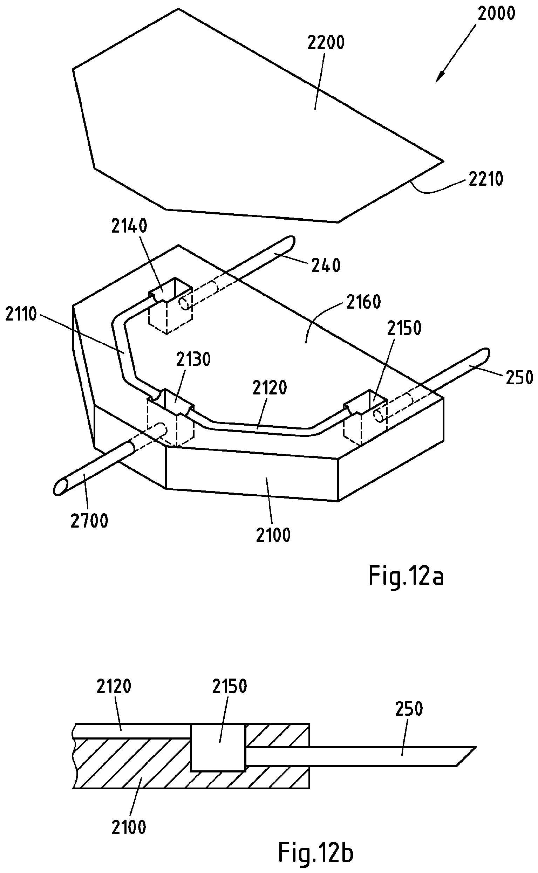

Fig. 12 to 13 illustrate an embodiment of a dispense interface 2000 alternative to the embodiment of the dispense interface 200 illustrated in Fig. 7 to 11. In Fig. 12 to 13 the same reference signs as in Fig. 7 to 11 are used for parts which are similar. Furthermore, at this point, it is mainly referred to the above description of the embodiment of the dispense interface 200 illustrated in Fig. 7 to 11 and, basically, the differences are described only.

As will now be discussed in greater detail, in one preferred arrangement, the dispense interface 2000 illustrated in Fig. 12 to 13 comprises:
a. a body portion 2100;
b. a cover film 2200;
c. a first piercing needle 240;
d. a second piercing needle 250;
e. an optional valve arrangement comprising a first flap valve 2600 and a second flap valve 2650; and
f. an injection needle 2700.

One exemplary difference between the dispense interface 200 and the dispense interface 2000 is the outer shape. Nevertheless, the dispense interface 2000 may comprise axial attachment means (not illustrated) and be attachable to a drug deliver device by such axial attachment means as described above with respect to dispense interface 200.

Fig. 12a illustrates a perspective view of an alternative embodiment of a dispense interface 2000 before bonding the cover film 2200 to the body portion 2100. The body portion 2100 comprises fluid grooves 2110 and 2120 arranged in the surface 2160 of the body portion 2100. The piercing needle 240 resides in fluid communication with the fluid groove 2110 via the ullage in form of a recess 2140, and the piercing needle 250 resides in fluid communication with the fluid groove 2120 via a second ullage in form of a recess 2150. The recesses 2140, 2150 each open out into the fluid grooves 2110 and 2120, respectively. The fluid grooves 2110, 2120 then converge to a third ullage in form of a recess 2130.

The recess 2130 resides in fluid communication with the injection needle 2700. Injection needle 2700 may be a cannula like needle 406. Alternatively, the recess 2130 may reside in fluid connection with a fluid connector (e.g. a pierceable septum 270) such that needle assembly 400 or any other standard needle assembly (not shown) may be attachable to the dispense interface 2000.

The dispense interface further comprises a cover film 2200. The cover film 2200 is not yet bonded to the body portion 2100 as illustrated in Fig. 12a. The cover film may be a polymer film or a laminate, for example. As can be seen in Fig. 12a, the cover film has substantially the same outer shape as the surface 2160 of the body portion 2100. However other shapes may be provided.

When the surface 2210 of the cover film 2200 facing the surface 2160 of the body portion 2100 is bonded to the surface 2160 of the body portion 2100, the fluid channels 2110, 2120 and the ullages 2130, 2140, 2150 are covered by the cover film and are thus sealed.

Fig. 12b illustrates an enlarged cross sectional view of the body portion 2100 of Fig. 12a, showing exemplarily the second injection needle 250, the second recess 2150 and the part of the fluid groove 2120. The piercing needle 250 is over-molded or potted such that it ends in the adjacent recess 2150. The recess 2150 is of rectangular design in this embodiment and opens out into the adjacent fluid groove 2120.

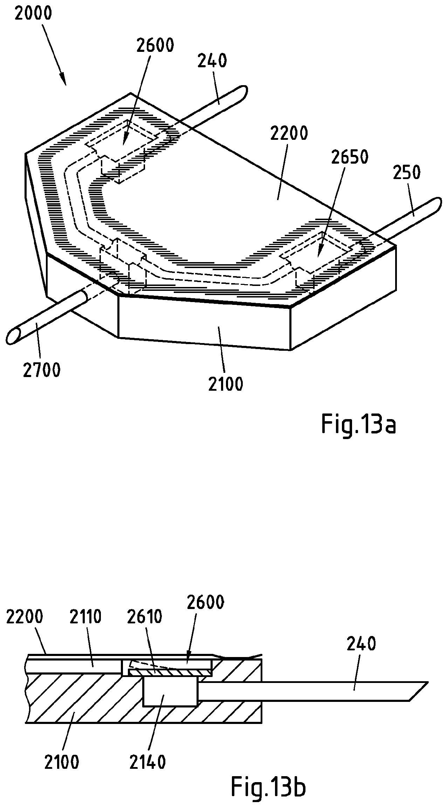

The dispense interface 2000 may optionally comprise a valve arrangement. Here, the recess 2150 does not comprise a valve, it may however serve as a housing or a chamber for a valve 2650. The recess 2140 likewise may serve as a housing or chamber for a valve 2600 (confer Fig. 13b). Alternatively or additionally, the recess 2130 may also comprise a valve.

Fig. 13a illustrates a perspective view of the dispense interface 2000 from Fig. 12a after bonding the cover film 2200 to the body portion 2100. When the body portion 2100 and the cover film 2200 are bonded together such that the surface 2160 of the body portion 2100 and the surface 2210 of the film cover face each other, the generally flat surface 2210 of the cover film seals the fluid grooves 2110 and 2120 and the recesses 2130, 2140, 2150 arranged in the surface 2160 of the body portion. The bonding may be realized by thermal or adhesive bonding techniques. In this embodiment, the bonding is only performed locally in the area of the fluid grooves 2110, 2120 and recesses 2130, 2140, 2150, as it is schematically illustrated by the shaded region in Fig. 13a. Alternatively, the bonding may be performed substantially in the area of the whole surface 2210 and/or 2160.

In this exemplary embodiment, before bonding of the cover film 2200 and the body portion 2100, a valve arrangement comprising the valves 2600, 2650 were positioned in the recesses 2140 and 2150, respectively.

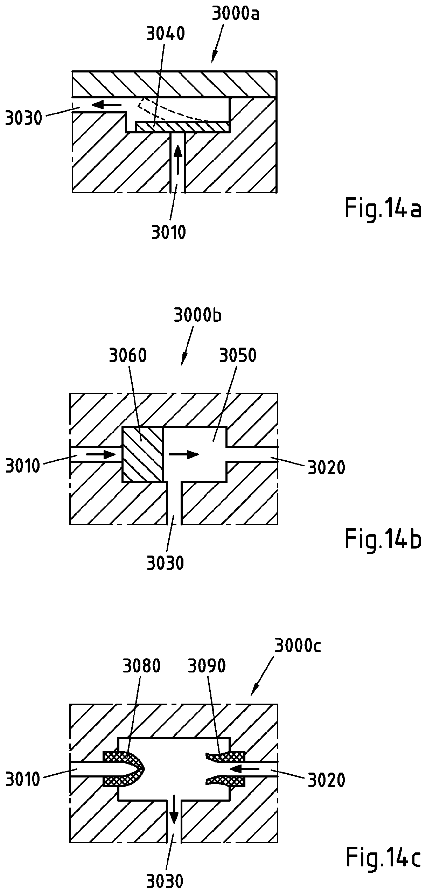

In Fig. 13b, exemplarily illustrating an enlarged cross sectional view of the ullage 2140 of the dispense interface 2000 from Fig. 13a, the optional valve 2600 with a flap 2610 is shown. The cover film 2200 is bonded to the body portion next to the recess 2140 for fluid tight sealing. Likewise, a valve is implemented in the recess 2650. However, the dispense interface may also comprise no valve arrangement (cf. Fig. 12b) or an alternative valve arrangement such as one of the embodiments illustrated in Fig. 14a to 14e.

The function of the optional first and second flap valves 2600, 2650 of the dispense interface 2000 may basically relate to the function of the first and second non return valve 262, 264 of the dispense interface 200. As described above, such a valve arrangement may for instance be constructed so as to prevent back flow and/or cross contamination of the first and second medicaments 92, 102 contained in the first and second reservoirs 90, 100, respectively.

As described in more detail with respect to Fig. 14a below, when the fluidic pressure in the needle 240 is increased (e.g. during a dose priming or a dose injecting step), the flap 2610 of valve 2600 will change from an un-stressed state to a stressed state. In this stressed condition, the fluidic pressure bends the flap 2610 (as indicated with the dashed line in Fig. 13b) and will allow fluid to flow from the piercing needle 240 to the injection needle 2700. When the fluidic pressure in the needle 240 is removed, the flap 2610 will return to its un-stressed state and seal the ullage 2140, preventing backflow. The flap valve 2650 operates in a similar manner as the flap valve 2600 when the fluidic pressure is increased in the needle 250.

Fig. 14a to 14e illustrate embodiments of a valve arrangement for a dispense interface alternative to the valve seal 260 of dispense interface 200 and duckbill valves 2600, 2650 of dispense interface 2000, respectively. In Fig. 14a to 14e the same reference signs are used for parts which are similar.

The valve arrangement may for instance be integrally formed with another part of the dispense interface. Alternatively, the valve arrangement may for instance be manufactured separately from the other parts of dispense interface.

For instance, the valve arrangement may be inserted (e.g. potted/over-molded/mounted) into the body portion. For instance, the valve arrangement may at least partially be potted/over-molded when the body portion is (e.g. injection) molded. For instance, the valve arrangement may at least partially be mounted in a separate step after the body portion has been (injection) molded.

Fig. 14a illustrates a diaphragm/flap valve arrangement 3000a. The diaphragm/flap valve arrangement 3000a has an inlet 3010 and an outlet 3030. The inlet 3010 may for instance reside in fluid communication with one of the piercing needles 240, 250 of dispense interface 200 or 2000, and the outlet 3030 may for instance reside in fluid communication with holding chamber 280 of dispense interface 200 or injection needle 2700 of dispense interface 2000.

The diaphragm/flap valve arrangement 3000a has a flexible diaphragm/flap 3040. When the fluidic pressure in the inlet 3010 is increased (e.g. during a dose priming or a dose injecting step), the diaphragm/flap 3040 will change from an un-stressed state to a stressed state. In the stressed state, the fluidic pressure bends the diaphragm/flap 3040 as indicated by the arrow in Fig. 14a so that the diaphragm/flap valve arrangement 3000a opens. In this stressed condition, the diaphragm/flap valve arrangement 3000a will allow fluid to flow from the inlet 3010 to the outlet 3030. When the fluidic pressure in the inlet is removed, the diaphragm/flap 3040 will return to its initial position and seal the inlet 3010, preventing backflow.

Fig. 14b illustrates a shuttling valve arrangement 3000b. The shuttling valve arrangement 3000b has a tube 3050. The tube 3050 has two inlets 3010, 3020 and an outlet 3030. The inlet 3020 may also reside in fluid communication with one of the piercing needles 240, 250 of dispense interface 200 or 2000. In the tube 3050 a movable element 3060 (e.g. a piston or a ball) is arranged.

The diameter of the movable element 3060 corresponds to the diameter of the tube 3050 such that the movable element 3060 is movable between a first and a second (longitudinal) position in the tube 3050. In the first position (illustrated in Fig. 14b), the movable element 3060 seals the inlet 3010 and allows fluid to flow from the inlet 3020 to the outlet 3030. In the second position (not illustrated), the movable element 3060 seals the inlet 3020 and allows fluid to flow from the inlet 3010 to the outlet 3030. When the fluidic pressure in the inlet 3010 is for instance increased (e.g. during a dose priming or a dose injecting step), the movable element 3060 will be pushed towards the second position as indicated by the arrow in Fig. 14b.

Fig. 14c illustrates a molded duckbill valve arrangement 3000c. The molded duckbill valve arrangement 3000c has a first and a second duckbill valve 3080, 3090. When the fluidic pressure in the inlet 3020 is increased (e.g. during a dose priming or a dose injecting step), the second duckbill valve 3090 will change from an un-stressed state to a stressed state. In the stressed state, the fluidic pressure inverts the naturally flattened shape of the duckbill valve as indicated in Fig. 14c so that the duckbill valve opens. In this stressed condition, the second duckbill valve 3090 will allow fluid to flow from the inlet 3020 to the outlet 3030. When the fluidic pressure in the inlet 3020 is removed, the second duckbill valve 3090 will return to its flattened shape and seal the inlet 3020, preventing backflow. The first duckbill valve 3080 operates in a similar manner as the second duckbill valve 3090 when the fluidic pressure is increased in the inlet 3010.

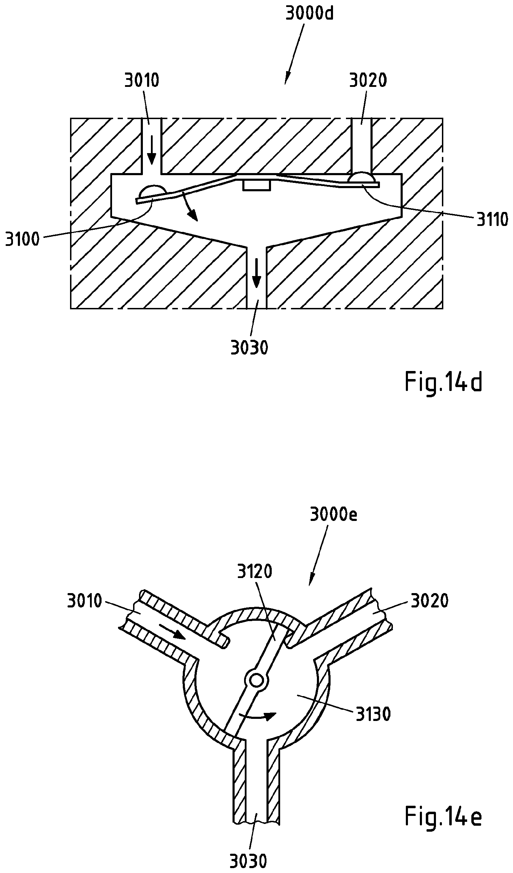

Fig. 14d illustrates a flat spring valve arrangement 3000d. The flat spring valve arrangement 3000d has a first and a second flat spring 3100, 3110. The first and the second flat spring 3100, 3110 may for instance be integrally formed.

When the fluidic pressure in the inlet 3010 is increased (e.g. during a dose priming or a dose injecting step), the first flat spring 3100 will change from an un-stressed state to a stressed state. In the stressed state, the fluidic pressure bends the first flat spring 3100 as indicated by the arrow in Fig. 14d so that the flat spring valve arrangement 3000d opens. In this stressed condition, the flat spring valve arrangement 3000d will allow fluid to flow from the inlet 3010 to the outlet 3030. When the fluidic pressure in the inlet 3010 is removed, the first flat spring 3100 will return to its initial position and seal the inlet 3010, preventing backflow. The second flat spring 3110 operates in a similar manner as the first flat spring 3100 when the fluidic pressure is increased in the inlet 3020.

Fig. 14e illustrates a rotating flap valve arrangement 3000e. The rotating flap valve arrangement 3000e has a flap 3120 which is rotatably mounted in a valve chamber 3130. The valve chamber has two inlets 3010, 3020 and an outlet 3030.

The flap 3120 is rotatable between a fist and a second position. In the first position (illustrated in Fig. 14e), the flap 3120 seals the inlet 3010 and allows fluid to flow from the inlet 3020 to the outlet 3030. In the second position (not illustrated), the flap 3120 seals the inlet 3020 and allows fluid to flow from the inlet 3010 to the outlet 3030. When the fluidic pressure in the inlet 3010 is for instance increased (e.g. during a dose priming or a dose injecting step), the flap 3120 will be pushed towards the second position as indicated by the arrow in Fig. 14e.

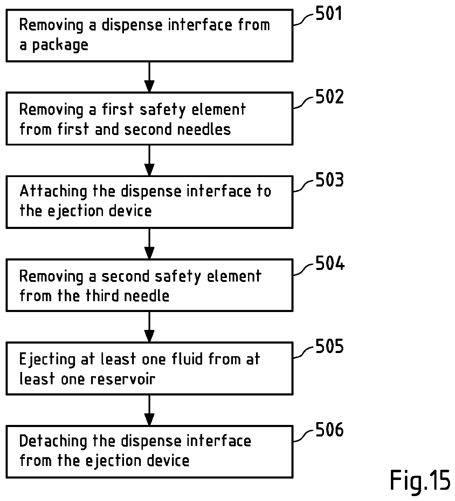

Fig. 15 illustrates a flowchart of a method according to the invention for using a dispense interface. In particular, the use of a previously described dispense interface is illustrated.

In a first step 501, a packaging of the dispense interface can be opened by a user and the dispense interface can be taken from the packaging.

Then, in step 502, if the dispense interface is provided with a first safety element, like a needle cover, the first safety element can be removed from the first proximal needle and/or the second proximal needle. For instance, if a predetermined breaking line is provided, the fist safety element can be detached by an angular movement performed by the user. It shall be understood that in alternative embodiments, the safety element can be formed by caps or the like.

After removing the first safety element, the first and second proximal needles are exposed. Then in step 503, the dispense interface is attached to an ejection device. In particular, the dispense interface is tightly attached to the ejection device. Thereby, the first proximal needle can puncture a first reservoir and the second proximal needle can puncture a second reservoir of the ejection device.

If the dispense interface comprises a second safety element for covering an ejection needle, in step 504, the second safety element is removed. The third needle, like an ejection needle, is exposed. For instance, if a predetermined breaking line is provided, the safety element can be removed by a circular and pull movement performed by the user. For avoiding a detachment of the dispense interface from the ejection device, the predetermined breaking line can be first cut by the circular movement and then the safety element can be removed by a pull movement.

In the next step 505, at least one fluid of at least one reservoir can be ejected, as described hereinbefore. For instance, a drug or medicament can be ejected.

Afterwards, the used dispense interface is detached from the ejection device (step 506). For instance, the used dispense interface can be pulled out by the user.

The term "drug" or "medicament", as used herein, means a pharmaceutical formulation containing at least one pharmaceutically active compound,

wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,

wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,

wherein in a further embodiment the pharmaceutically active compound comprises at least one peptide for the treatment and/or prophylaxis of diabetes mellitus or complications associated with diabetes mellitus such as diabetic retinopathy,

wherein in a further embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyhepta¬decanoyl) human insulin.

Exendin-4 for example means Exendin-4(1-39), a peptide of the sequence H His-Gly-Glu-Gly-Thr-Phe-Thr-Ser-Asp-Leu-Ser-Lys-Gln-Met-Glu-Glu-Glu-Ala-Val-Arg-Leu-Phe-lle-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39); or
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39),
wherein the group -Lys6-NH2 may be bound to the C-terminus of the Exendin-4 derivative;
or an Exendin-4 derivative of the sequence
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(O2)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(O2)25, Asp28] Exendin-4(1-39)-NH2,
des Pro36, Pro37, Pro38 [Trp(O2)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(O2)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(O2)25, Asp28] Exendin-4(1-39)-Lys6-NH2,
H-des Asp28 Pro36, Pro37, Pro38 [Met(O)14, Trp(O2)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(O2)25, Asp28] Exendin-4(1-39)-NH2,
des Pro36, Pro37, Pro38 [Met(O)14, Trp(O2)25, Asp28] Exendin-4(1-39)-(Lys)6-NH2,
H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (-150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (CH) and the variable region (VH). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCI or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1 C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

## Claims

1. Dispense interface for an ejection device (10), said dispense interface (2000) being attachable to said ejection device and comprising:
- at least two inlets (240, 250);
- at least one outlet (2700);
- a body portion (2100); and wherein
- the dispense interface further comprises a cover film (2200);
- wherein said body portion (2100) and said cover film (2200) are bonded together;
- wherein said body portion (2100) and said cover film (2200) are configured to form a fluid channel arrangement between surfaces (2160, 2210) of said body portion (2100) and said cover film (2200) facing each other when said body portion (2100) and said cover film (2200) are bonded together;
- wherein the fluid channel arrangement comprises fluid grooves (2110, 2120) **characterized in that**, the fluid grooves (2110, 2120) are arranged in said surface (2120) of said body portion (2100), wherein the surface (2210) of the cover film (2200) seals the fluid grooves (2110, 2120) when the body portion (2100) and the cover film (2200) are bonded together,
- wherein said fluid channel arrangement is configured to provide fluid communication between said at least two inlets (240, 250) and said at least one outlet (2700),
- wherein each of said at least two inlets is formed from a fluid connector (240, 250) emptying into said fluid channel arrangement, and wherein each of said fluid connectors (240, 250) is configured to establish a releasable fluid connection with a corresponding fluid connector of a fluid reservoir (90, 100) of said ejection device (10) when said dispense interface (2000) is attached to said ejection device.

2. Dispense interface according to claim 1, wherein said cover film (2200) is a metal film, a polymer film, or a bio-polymer film.

3. Dispense interface according to claim 1 or 2, wherein said body portion (2100) is manufactured from a polymer material.

4. Dispense interface according to any of claims 1 and 3, wherein said fluid connectors (240, 250) forming each of said at least two inlets are piercing needles, in particular at least partially inserted into the body portion (2100).

5. Dispense interface according to any of claims 1 to 4, wherein said at least one outlet is formed from a fluid connector, wherein said fluid channel arrangement empties into said fluid connector, and wherein said fluid connector is configured to establish a fluid connection with a corresponding fluid connector of a needle assembly (400), when said needle assembly is attached to said dispense interface (2000).

6. Dispense interface according to any of claims 1 to 5, wherein said at least one outlet is formed from a needle (2700), wherein said fluid channel arrangement empties into said needle (2700).

7. Dispense interface according to any preceding claim, wherein the surface (2210) of the cover film (220) that seals the fluid grooves (2110, 2120) is generally flat.

8. Dispense interface according to any preceding claim, said dispense interface (2000) further comprising:
- at least one ullage (2130, 2140, 2150);
- wherein said ullage (2130, 2140, 2150) provides fluid communication between an adjacent fluid groove (2110, 2120) and an adjacent inlet (240, 250) or outlet (2700).

9. Dispense interface according to any of claims 1 to 8, said dispense interface (2000) further comprising:
- a valve arrangement (2600, 2650, 3000a, 3000b, 3000c, 3000d, 3000e) configured to control a fluid flow from said at least two inlets (240, 250) to said at least one outlet (2700) via said fluid channel arrangement.

10. Method for manufacturing a dispense interface according to any of the claims 1 to 9, said method comprising:
- molding fluid grooves (2110, 2120) into a surface (2160) of a body portion (2100);
- bonding a cover film (2200) to the body portion (2100) such that a fluid channel arrangement is formed between surfaces (2160, 2210) of said body portion (2100) and said cover film (2200) facing each other when said body portion (2100) and said cover film (2200) are bonded together,
- wherein the surface (2210) of the cover film (2200) seals the fluid grooves (2110, 2120) when the body portion (2100) and the cover film (2200) are bonded together.

11. Method according to claim 10, said method further comprising:
- connecting fluid connectors (240, 250, 2700) forming the at least two inlets and the at least one outlet to the body portion in a sealing manner.

12. System, comprising
- a dispense interface (2000) according to any of claims 1 to 9; and
- an ejection device (10);
- wherein said dispense interface (2000) is attached to said ejection device (10).

13. System according to claim 12, said system further comprising:
- a needle assembly (400);
- wherein said needle assembly (400) is attached to said dispense interface (2000).

14. System according to any of claims 12 and 13, wherein said ejection device (10) is a medical device configured to eject a medicament (92, 102).

15. Method for using a system according to any of the claims 12 to 14, excluding in-vivo applications, said method comprising:
- attaching said dispense interface (2000) to an ejection device (10) having at least two fluid reservoirs (90, 100);
- ejecting a fluid (92, 102) from at least one of the reservoirs (90, 100) through said dispense interface (2000); and
- detaching said dispense interface (2000) from said ejection device (10).

## Patentansprüche

1. Abgabeschnittstelle für eine Ausstoßvorrichtung (10), wobei die Abgabeschnittstelle (2000) an der Ausstoßvorrichtung anbringbar ist und Folgendes umfasst:
- mindestens zwei Einlässe (240, 250),
- mindestens zwei Auslässe (2700),
- einen Körperabschnitt (2100) und
wobei
- die Abgabeschnittstelle ferner eine Deckfolie (2200) umfasst,
- wobei der Körperabschnitt (2100) und die Deckfolie (2200) aneinander gebondet sind,
- wobei der Körperabschnitt (2100) und die Deckfolie (2200) ausgebildet sind, um eine Fluidkanalanordnung zwischen Flächen (2160, 2210) des Körperabschnitts (2100) und der Deckfolie (2200), die einander gegenüberliegen, wenn der Körperabschnitt (2100) und die Deckfolie (2200) aneinander gebondet sind, zu bilden,
- wobei die Fluidkanalanordnung Fluidnuten (2110, 2120) umfasst,
**dadurch gekennzeichnet, dass** die Fluidnuten (2110, 2120) in der Fläche (2120) des Körperabschnitts (2100) angeordnet sind, wobei die Fläche (2210) der Deckfolie (2200) die Fluidnuten (2110, 2120) abdichtet, wenn der Körperabschnitt (2100) und die Deckfolie (2200) aneinander gebondet sind,
- wobei die Fluidkanalanordnung ausgebildet ist, eine Fluidverbindung zwischen den mindestens zwei Einlässen (240, 250) und dem mindestens einen Auslass (2700) bereitzustellen,
- wobei jeder der mindestens zwei Einlässe aus einem Fluidverbinder (240, 250) gebildet ist, der sich in die Fluidkanalanordnung entleert, und wobei jeder der Fluidverbinder (240, 250) ausgebildet ist, eine freigebbare Fluidverbindung zu einem entsprechenden Fluidverbinder eines Fluidreservoirs (90, 100) der Ausstoßvorrichtung (10) herzustellen, wenn die Abgabeschnittstelle (2000) an der Ausstoßvorrichtung angebracht ist.

2. Abgabeschnittstelle nach Anspruch 1, wobei die Deckfolie (2200) eine Metallfolie, eine Polymerfolie oder eine Biopolymerfolie ist.

3. Abgabeschnittstelle nach Anspruch 1 oder 2, wobei der Körperabschnitt (2100) aus einem Polymermaterial hergestellt ist.

4. Abgabeschnittstelle nach einem der Ansprüche 1 und 3, wobei die jeden der mindestens zwei Einlässe bildenden Fluidverbinder (240, 250) Einstechnadeln sind, die insbesondere mindestens teilweise in den Körperabschnitt (2100) eingeführt sind.

5. Abgabeschnittstelle nach einem der Ansprüche 1 bis 4, wobei der mindestens eine Auslass aus einem Fluidverbinder gebildet ist, wobei sich die Fluidkanalanordnung in den Fluidverbinder entleert und wobei der Fluidverbinder ausgebildet ist, eine Fluidverbindung mit einem entsprechenden Fluidverbinder einer Nadelanordnung (400) herzustellen, wenn die Nadelanordnung an der Abgabeschnittstelle (2000) angebracht ist.

6. Abgabeschnittstelle nach einem der Ansprüche 1 bis 5, wobei der mindestens eine Auslass aus einer Nadel (2700) gebildet ist, wobei sich die Fluidkanalanordnung in die Nadel (2700) entleert.

7. Abgabeschnittstelle nach einem der vorhergehenden Ansprüche, wobei die die Fluidnuten (2110, 2120) abdichtende Fläche (2210) der Deckfolie (220) allgemein flach ist.

8. Abgabeschnittstelle nach einem der vorhergehenden Ansprüche, wobei die Abgabeschnittstelle (2000) ferner Folgendes umfasst:
- mindestens eine Ullage (2130, 2140, 2150),
- wobei die Ullage (2130, 2140, 2150) eine Fluidverbindung zwischen einer benachbarten Fluidnut (2110, 2120) und einem benachbarten Einlass (240, 250) oder Auslass (2700) bereitstellt.

9. Abgabeschnittstelle nach einem der Ansprüche 1 bis 8, wobei die Abgabeschnittstelle (2000) ferner Folgendes umfasst:
- eine Ventilanordnung (2600, 2650, 3000a, 3000b, 3000c, 3000d, 3000e), die ausgebildet ist, um eine Fluidströmung von den mindestens zwei Einlässen (240, 250) zu dem mindestens einen Auslass (2700) über die Fluidkanalanordnung zu steuern.

10. Verfahren zur Herstellung einer Abgabeschnittstelle nach einem der Ansprüche 1 bis 9, wobei das Verfahren Folgendes umfasst:
- Formen von Fluidnuten (2110, 2120) in eine Fläche (2160) eines Körperabschnitts (2100),
- Bonden einer Deckfolie (2200) an den Körperabschnitt (2100), so dass eine Fluidkanalanordnung zwischen Flächen (2160, 2210) des Körperabschnitts (2100) und der Deckfolie (2200), die einander gegenüberliegen, wenn der Körperabschnitt (2100) und die Deckfolie (2200) aneinander gebondet sind, gebildet wird,
- wobei die Fläche (2210) der Deckfolie (2200) die Fluidnuten (2110, 2120) abdichtet, wenn der Körperabschnitt (2100) und die Deckfolie (2200) aneinander gebondet sind.

11. Verfahren nach Anspruch 10, wobei das Verfahren ferner Folgendes umfasst:
- dichtendes Verbinden der die mindestens zwei Einlässe und den mindestens einen Auslass bildenden Fluidverbinder (240, 250, 2700) mit dem Körperabschnitt.

12. System, umfassend:
- eine Abgabeschnittstelle (2000) nach einem der Ansprüche 1 bis 9 und
- eine Ausstoßvorrichtung (10),
- wobei die Abgabeschnittstelle (2000) an der Ausstoßvorrichtung (10) angebracht ist.

13. System nach Anspruch 12, wobei das System ferner Folgendes umfasst:
- eine Nadelanordnung (400),
- wobei die Nadelanordnung (400) an der Abgabeschnittstelle (2000) angebracht ist.

14. System nach einem der Ansprüche 12 und 13, wobei die Ausstoßvorrichtung (10) eine zum Ausstoßen eines Medikaments (92, 102) ausgebildete medizinische Vorrichtung ist.

15. Verfahren zur Verwendung eines Systems nach einem der Ansprüche 12 bis 14, ausschließlich In-vivo-Anwendungen, wobei das Verfahren Folgendes umfasst:
- Anbringen der Abgabeschnittstelle (2000) an einer Ausstoßvorrichtung (10) mit mindestens zwei Fluidreservoirs (90, 100),
- Ausstoßen eines Fluids (92, 102) aus mindestens einem der Reservoirs (90, 100) durch die Abgabeschnittstelle (2000) und
- Ablösen der Abgabeschnittstelle (2000) von der Ausstoßvorrichtung (10).

## Revendications

1. Interface de distribution pour un dispositif d'éjection (10), ladite interface de distribution (2000) étant apte à être fixée audit dispositif d'éjection et comprenant :
- au moins deux entrées (240, 250) ;
- au moins une sortie (2700) ;
- une partie corps (2100) ; et
- l'interface de distribution comprenant en outre un film de revêtement (2200) ;
- ladite partie corps (2100) et ledit film de revêtement (2200) étant liés l'un à l'autre ;
- ladite partie corps (2100) et ledit film de revêtement (2200) étant configurés pour former une structure de canal à fluide entre des surfaces (2160, 2210) de ladite partie corps (2100) et dudit film de revêtement (2200) se faisant face lorsque ladite partie corps (2100) et ledit film de revêtement (2200) sont liés l'un à l'autre ;
- la structure de canal à fluide comprenant des rainures à fluide (2110, 2120),
**caractérisée en ce que** les rainures à fluide (2110, 2120) sont placées dans ladite surface (2120) de ladite partie corps (2100), la surface (2210) du film de revêtement (2200) fermant hermétiquement les rainures à fluide (2110, 2120) lorsque la partie corps (2100) et le film de revêtement (2200) sont liés l'un à l'autre,
- ladite structure de canal à fluide étant configurée pour assurer une communication fluidique entre lesdites au moins deux entrées (240, 250) et ladite au moins une sortie (2700),
- chacune desdites au moins deux entrées étant constituée d'un organe de liaison fluidique (240, 250) débouchant dans ladite structure de canal à fluide, et chacun desdits organes de liaison fluidique (240, 250) étant configuré pour établir une liaison fluidique interruptible avec un organe de liaison fluidique correspondant d'un réservoir à fluide (90, 100) dudit dispositif d'éjection (10) lorsque ladite interface de distribution (2000) est fixée audit dispositif d'éjection.

2. Interface de distribution selon la revendication 1, dans laquelle ledit film de revêtement (2200) est un film de métal, un film de polymère ou un film de biopolymère.

3. Interface de distribution selon la revendication 1 ou 2, dans laquelle ladite partie corps (2100) est fabriquée à partir d'un matériau polymère.

4. Interface de distribution selon l'une quelconque des revendications 1 à 3, dans laquelle lesdits organes de liaison fluidique (240, 250) formant chacune desdites au moins deux entrées sont des aiguilles de perforation, en particulier au moins partiellement insérées dans la partie corps (2100) .

5. Interface de distribution selon l'une quelconque des revendications 1 à 4, dans laquelle ladite au moins une sortie est constituée d'un organe de liaison fluidique, dans laquelle ladite structure de canal à fluide débouche dans ledit organe de liaison fluidique, et dans laquelle ledit organe de liaison fluidique est configuré pour établir une liaison fluidique avec un organe de liaison fluidique correspondant d'un ensemble formant aiguille (400), lorsque ledit ensemble formant aiguille est fixé à ladite interface de distribution (2000).

6. Interface de distribution selon l'une quelconque des revendications 1 à 5, dans laquelle ladite au moins une sortie est constituée d'une aiguille (2700), dans laquelle ladite structure de canal à fluide débouche dans ladite aiguille (2700).

7. Interface de distribution selon l'une quelconque des revendications précédentes, dans laquelle la surface (2210) du film de revêtement (220) qui ferme hermétiquement les rainures à fluide (2110, 2120) est globalement plane.

8. Interface de distribution selon l'une quelconque des revendications précédentes, ladite interface de distribution (2000) comprenant en outre :
- au moins une cavité (2130, 2140, 2150) ;
- dans laquelle ladite cavité (2130, 2140, 2150) assure une communication fluidique entre une rainure à fluide (2110, 2120) adjacente et une entrée (240, 250) ou une sortie (2700) adjacente.

9. Interface de distribution selon l'une quelconque des revendications 1 à 8, ladite interface de distribution (2000) comprenant en outre :
- une structure de valve (2600, 2650, 3000a, 3000b, 3000c, 3000d, 3000e) configurée pour réguler un écoulement de fluide desdites au moins deux entrées (240, 250) à ladite au moins une sortie (2700) passant par ladite structure de canal à fluide.

10. Procédé de fabrication d'une interface de distribution selon l'une quelconque des revendications 1 à 9, ledit procédé comprenant :
- mouler des rainures à fluide (2110, 2120) dans une surface (2160) d'une partie corps (2100) ;
- lier un film de revêtement (2200) à la partie corps (2100) de telle sorte qu'une structure de canal à fluide soit formée entre des surfaces (2160, 2210) de ladite partie corps (2100) et dudit film de revêtement (2200) se faisant face lorsque ladite partie corps (2100) et ledit film de revêtement (2200) sont liés l'un à l'autre,
- dans lequel la surface (2210) du film de revêtement (2200) ferme hermétiquement les rainures à fluide (2110, 2120) lorsque la partie corps (2100) et le film de revêtement (2200) sont liés l'un à l'autre.

11. Procédé selon la revendication 10, ledit procédé comprenant en outre :
- relier des organes de liaison fluidique (240, 250, 2700) formant les au moins deux entrées et l'au moins une sortie à la partie corps de manière étanche.

12. Système comprenant :
- une interface de distribution (2000) selon l'une quelconque des revendications 1 à 9 ; et
- un dispositif d'éjection (10) ;
- dans lequel ladite interface de distribution (2000) est fixée audit dispositif d'éjection (10).

13. Système selon la revendication 12, ledit système comprenant en outre :
- un ensemble formant aiguille (400) ;
- dans lequel ledit ensemble formant aiguille (400) est fixé à ladite interface de distribution (2000) .

14. Système selon l'une quelconque des revendications 12 et 13, dans lequel ledit dispositif d'éjection (10) est un dispositif médical configuré pour éjecter un médicament (92, 102).

15. Procédé d'utilisation d'un système selon l'une quelconque des revendications 12 à 14, à l'exclusion des applications *in vivo,* ledit procédé comprenant :
- fixer ladite interface de distribution (2000) à un dispositif d'éjection (10) comportant au moins deux réservoirs à fluide (90, 100) ;
- éjecter un fluide (92, 102) à partir d'au moins un des réservoirs (90, 100) à travers ladite interface de distribution (2000) ; et
- détacher ladite interface de distribution (2000) dudit dispositif d'éjection (10).
